(19) Europäisches Patentamt
European Patent Office
Office européen des brevets

(11) Veröffentlichungsnummer : **0 022 972**
**B1**

(12) **EUROPÄISCHE PATENTSCHRIFT**

(45) Veröffentlichungstag der Patentschrift : **26.01.83**

(21) Anmeldenummer : **80103853.0**

(22) Anmeldetag : **07.07.80**

(51) Int. Cl.³ : **C 07 C 69/743**, C 07 B 19/00, C 07 C 61/16, C 07 C 67/08, C 07 C 67/30, C 07 C 51/493// A01 N53/00

(54) **Neue Menthylester, Verfahren zu ihrer Herstellung und ihre Verwendung zur Enantiomerentrennung chiraler Carbonsäuren.**

(30) Priorität : **13.07.79 DE 2928406**

(43) Veröffentlichungstag der Anmeldung : **28.01.81 Patentblatt 81/04**

(45) Bekanntmachung des Hinweises auf die Patenterteilung : **26.01.83 Patentblatt 83/04**

(84) Benannte Vertragsstaaten :
**BE CH DE FR GB IT LI NL**

(56) Entgegenhaltungen :
**DE A 2 718 039**
**DE B 2 240 257**

**JOURNAL OF AGRICULTURE & FOOD CHEMISTRY, Band 24, Nr. 2, 1976 M. ELLIOTT et al. « Radiosynthesis and Metabolism in Rats of the 1R-Isomers of the Insecticide Permethrin » Seiten 270 to 276.**

(73) Patentinhaber : **BAYER AG**
**Zentralbereich Patente, Marken und Lizenzen**
**D-5090 Leverkusen 1, Bayerwerk (DE)**

(72) Erfinder : **Naumann, Klaus, Dr.**
**Richard-Wagner-Strasse 9**
**D-5090 Leverkusen 1 (DE)**
Erfinder : **Rauchschwalbe, Rudolf, Dr.**
**Pahlkestrasse 5**
**D-5600 Wuppertal (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

Neue Menthylester, Verfahren zu ihrer Herstellung und ihre Verwendung zur Enantiomerentrennung chiraler Carbonsäuren

Die vorliegende Erfindung betrifft neue Menthylester substituierter Cyclopropancarbonsäuren, Verfahren zu ihrer Herstellung und ihre Verwendung zur Trennung der Enantiomeren dieser Carbonsäuren.

Aus J. Agric. Food. Chem. Vol. 24, No 2, 1976, Seiten 270ff sind die l-Menthylester von 1R/1S cis, 1R/1S trans, 1R cis, 1S cis 3-(2,2-Dichlorvinyl-)2,2-dimethyl-cyclopropancarbonsäure (Permethrinsäure) bekannt. Die Verbindungen werden durch Umsetzung von l-Menthol mit dem entsprechenden Isomeren bzw. Isomerengemisch des Säurechlorids hergestellt. Die Verbindungen wurden zur Untersuchung der cis-trans-Epimerisierung der Permethrinsäure hergestellt. Angaben über eine technische Verwendung dieser Verbindungen liegen nicht vor. Auch werden keine Angaben über Herstellung und Verwendung der übrigen Menthylesterisomeren der Permethrinsäure gemacht.

Die substituierten Cyclopropancarbonsäuren der Formel besitzen zwei Asymmetriezentren (① und ②).

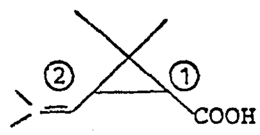

Zwischen beiden Zentren besteht außerdem die Möglichkeit einer cis/trans Isomerie. Um die sterischen Verhältnisse zu charakterisieren wird im folgenden die absolute Konfiguration des Asymmetriezentrums ① und zusätzlich die Isomerieverhältnisse entlang der Bindung ①-② angegeben. Durch diese Bezeichnung ist auch die absolute Konfiguration am Asymmetriezentrum 2 festgelegt. Die absolute Konfiguration wird mit den Buchstaben R und S bezeichnet, entsprechend der Nomenklatur von Cahn, Ingold, Prelog. Angew. Chemie 78, 413 (1966).

Die Buchstaben R bzw. S bezeichnen absolute Konfiguration der bezeichneten C-Atome der benannten Carbonsäuren. Die 1R Formen entsprechen den (+)-Enantiomeren, 1RS den Racematen.

Gegenstand der vorliegenden Erfindung sind

1) Neue d-Menthylester von 1RS cis, 1RS trans, 1RS cis/trans, 1R cis, 1S cis, 1R trans, 1S trans, 1R cis/trans, 1S cis/trans 3-(2,2-Dichlor-vinyl-)2,2-dimethyl-cyclopropancarbonsäure, neue d-Menthylester von 1RS cis 1RS trans, 1RS cis/trans, 1R cis, 1S cis, 1R trans, 1S trans, 1R cis/trans, 1S cis/trans 3-(2,2-Dibromvinyl-)2,2-dimethylcyclopropancarbonsäure sowie neue l-Menthylester von 1RS cis, 1RS trans, 1RS cis/trans, 1R cis, 1S cis, 1R trans, 1S trans, 1S cis/trans, 1R cis/trans 3-(2,2-Dibrom-vinyl-)2,2-dimethyl-cyclopropancarbonsäure sowie neue l-Menthylester von 1R trans, 1S cis/trans, 1R cis/trans, 1S trans 3-(2,2-Dichlor-vinyl)-2,2-dimethylcyclopropancarbonsäure.

2) Verfahren zur Herstellung der neuen Diastereomeren bzw. Diastereomerengemische von 3-(2,2-Dichlorvinyl-)2,2-dimethyl-cyclopropancarbonsäure und 3-(2,2-Dibromvinyl)-2,2-dimethylcyclopropancarbonsäure -d- bzw. l-Menthylester gemäß Anspruch 1, dadurch gekennzeichnet, daß man

a) die entsprechenden Säuren bzw. deren reaktionsfähige Derivate mit d- bzw. l-Menthol umsetzt, oder

b) Menthylester der Formel I

$$X_2C{=}CH{-}\underset{X}{CH}{-}\overset{CH_3}{\underset{CH_3}{C}}{-}CH_2COO\ Men \qquad (I)$$

in welcher

X für Chlor oder Brom steht und

Men für den l- oder d-Menthylrest steht der cyclisierden Dehydrohalogenierung unterwirft oder

c) Menthylester der Formel II

$$X_2C{=}CH{-}\underset{(CH_3)_2CX}{CH}{-}CH_2{-}COO\ Men$$

in welcher

X und Men die oben angegebene Bedeutung haben der cyclisierenden Dehydrohalogenierung unterwirft oder

d) Diazoessigsäure -d- bzw. -l-menthylester mit 1,1-Dichlor-4-methyl-pentadien-1,3 in Gegenwart von Kupferverbindungen umsetzt oder

e) für den Fall, daß man die d-Menthylester der 1R cis Säuren erhalten will, das Gemisch der entsprechenden 1RS cis-Säuren oder deren reaktionsfähigen Derivaten mit d-Menthol umsetzt und aus Alkanen, Alkoholen, wäßrigen Alkoholen oder Ketonen den schwerer löslichen 1R cis d-Menthylester in üblicher Weise abtrennt, oder

f) für den Fall, daß man die l-Menthylester der 1Scis-Säuren erhalten will, das Gemisch der entsprechenden 1RScis-Säuren oder deren reaktionsfähigen Derivaten mit l-Menthol umsetzt und aus Alkanen, Alkoholen, wäßrigen Alkoholen oder Ketonen den schwerer löslichen l-Menthylester der 1Scis-Säure in üblicher Weise abtrennt, oder

g) für den Fall, daß man die d-Menthylester der 1R-trans-Säure erhalten will, das Gemisch der entsprechenden 1RS trans-Säuren oder das Gemisch der entsprechenden 1RS cis/trans-Säuren oder deren reaktionsfähigen Derivaten mit d-Menthol umsetzt oder die Reaktionen gemäß b, c oder d (oben) mit den entsprechenden d-Menthylestern durchführt und aus Alkanen, Alkoholen, wäßrigen Alkoholen oder Ketonen den schwerer löslichen d-Menthylester der 1R trans-Säure in üblicher Weise abtrennt oder

h) für den Fall, daß man die l-Menthylester der 1S trans-Säure erhalten will, das Gemisch der entsprechenden 1RS trans-Säuren oder das Gemisch der entsprechenden 1RS cis/trans-Säuren oder deren reaktionsfähigen Derivaten mit l-Menthol umsetzt oder die Reaktionen gemäß b, c oder d (oben) mit den entsprechenden l-Menthylestern durchführt und aus Alkanen, Alkoholen, wäßrigen Alkoholen oder Ketonen den schwerer löslichen l-Menthylester der 1S trans-Säure in üblicher Weise abtrennt.

i) für den Fall, daß man das Gemisch der d-Menthylester der 1R-cis/trans-Säure erhalten will, das Gemisch der entsprechenden 1RS cis/trans-Säuren oder deren reaktionsfähigen Derivaten mit d-Menthol umsetzt oder die Reaktionen gemäß b, c oder d (oben) mit den entsprechenden d-Menthylestern durchführt und aus Ligroin die schwerer löslichen d-Menthylester der 1R-cis/trans-Säure in üblicher Weise abtrennt oder

k) für den Fall, daß man das Gemisch der l-Menthylester der 1R-cis/trans bzw. 1Scis/trans Säure erhalten will, das Gemisch der entsprechenden 1RS cis/trans-Säuren oder deren reaktionsfähigen Derivaten mit l-Menthol umsetzt oder die Reaktionen gemäß b, c oder d (oben) mit den entsprechenden l-Menthylestern durchführt und aus Ligroin den schwerer löslichen l-Menthylester der 1S cis/trans-Säure in üblicher Weise abtrennt und die leichter löslichen 1R cis/trans-Ester weiterverwendet.

3) Verfahren zur Abtrennung von 1R cis 3-(2,2-Dichlorvinyl-)2,2-dimethyl-cyclopropancarbonsäure oder 1R cis 3-(2,2-Dibromvinyl-)2,2-dimethyl-cyclopropancarbonsäure aus dem Gemisch der entsprechenden 1RS cis-Säuren, dadurch gekennzeichnet, daß man das Gemisch dieser Säuren oder ihrer reaktionsfähigen Derivate mit d-Menthol umsetzt, den in Alkanen, Alkoholen, wäßrigen Alkoholen oder Ketonen schwerer löslichen d-Menthylester der 1R cis-Säure abtrennt und die 1R cis-Säure durch Verseifung des Esters freisetzt.

4) Verfahren zur Abtrennung von 1S cis 3-(2,2-Dichlorvinyl-)2,2-dimethyl-cyclopropancarbonsäure oder 1S cis 3-(2,2-Dibromvinyl)-2,2-dimethyl-cyclopropancarbonsäure aus dem Gemisch der entsprechenden 1RS cis-Säuren, dadurch gekennzeichnet, daß man das Gemisch dieser Säuren oder ihrer reaktionsfähigen Derivate mit l-Menthol umsetzt, den in Alkanen, Alkoholen, wäßrigen Alkoholen oder Ketonen schwerer löslichen l-Menthylester der 1S cis-Säure abtrennt und die 1S cis-Säure durch Verseifung des Esters freisetzt.

5) Verfahren zur Abtrennung von 1R trans 3-(2,2-Dichlorvinyl-)2,2-dimethyl-cyclopropancarbonsäure oder 1R trans 3-(2,2-Dibromvinyl)-2,2-dimethyl-cyclopropancarbonsäure aus dem Gemisch der entsprechenden 1RS trans-Säuren oder dem Gemisch der entsprechenden 1RS cis/trans-Säuren oder den entsprechenden d-Menthylestergemischen, dadurch gekennzeichnet, daß man das Gemisch dieser Säuren oder ihrer reaktionsfähigen Derivate mit d-Menthol umsetzt, oder die Reaktionen gemäß 2b, c oder d (oben) mit den entsprechenden d-Menthylestern durchführt, den in Alkanen, Alkoholen, wäßrigem Alkoholen oder Ketonen schwerer löslichen d-Menthylester der 1R trans-Säure abtrennt und die 1R-trans-Säure durch Verseifung des Esters freisetzt.

6) Verfahren zur Abtrennung von 1S trans 3-(2,2-Dichlorvinyl-)2,2-dimethyl-cyclopropancarbonsäure oder 1S trans 3-(2,2-Dibromvinyl)-2,2-dimethyl-cyclopropancarbonsäure aus dem Gemisch der entsprechenden 1RS trans-Säuren oder dem Gemisch der entsprechenden 1RS cis/trans-Säuren oder den entsprechenden Menthylestergemischen, dadurch gekennzeichnet, daß man das Gemisch dieser Säuren oder ihrer reaktionsfähigen Derivate mit l-Menthol umsetzt oder die Reaktionen gemäß 2b, c oder d (oben) mit den entsprechenden Menthylestern durchführt, den in Alkanen, Alkoholen, wäßrigen Alkoholen oder Ketonen schwerer löslichen l-Menthylester der 1S trans-Säure abtrennt und die 1S trans-Säure durch Verseifung des Esters freisetzt.

7) Verfahren zur Abtrennung von 1R cis/trans 3-(2,2-Dichlorvinyl-)2,2-dimethyl-cyclopropancarbonsäure oder 1R cis/trans 3-(2,2-Dibromvinyl)-2,2-dimethylcyclopropancarbonsäure aus dem Gemisch der entsprechenden 1RS cis/trans-Säuren, dadurch gekennzeichnet, daß man das Gemisch dieser Säuren oder ihrer reaktionsfähigen Derivate mit d-Menthol umsetzt, die in Ligroin schwerer löslichen d-Menthylester der 1R cis/trans-Säure abtrennt und die 1R cis/trans Säure durch Verseifung des Esters freisetzt.

8) Verfahren zur Abtrennung von 1R cis/trans 3-(2,2-Dichlorvinyl-)2,2-dimethyl-cyclopropancarbonsäure oder 1R cis/trans 3-(2,2-Dibromvinyl)-2,2-dimethylcyclopropancarbonsäure aus dem Gemisch der entsprechenden 1RS cis/trans-Säuren oder den entsprechenden l-Menthylestergemischen,

dadurch gekennzeichnet, daß man das Gemisch dieser Säuren oder ihrer reaktionsfähigen Derivate mit l-Menthol umsetzt, oder die Reaktionen gemäß 2b, c oder d (oben) mit den entsprechenden l-Menthylestern durchführt, den in Ligroin schwerer löslichen l-Menthylester der 1S cis/trans-Säure abtrennt und die 1R cis/trans-Säure durch Verseifung des leicht löslichen Esters freisetzt.

Die neuen Menthylester lassen sich somit auf technisch einfache Weise zur Trennung der Enantiomeren von 3-(2,2-Dichlor- bzw. Dibromvinyl-)-2,2-dimethyl-cyclopropancarbonsäuren einsetzen. Dies war erstaunlich, da sich 3-(2-Methyl-propen-1-yl)-2,2-dimethyl-cyclopropancarbonsäure nicht über ihre entsprechenden Menthylester in die Enantiomeren spalten läßt.

Die Trennung der Enantiomeren dieser Säuren erfolgte bis jetzt durch fraktionierte Ausfällung bzw. Kristallisation der diastereomeren Salze der freien Säuren mit optisch aktiven Aminen (siehe z.B. Dervent Basical Abstr. Journal 45194 W/27, 21671 W/13, 13445 W/08 ; DE-OS 2549177 ; Franz. Pat. 1536468). Diese Verfahren sind für eine Anwendung im großen Maßstab umständlich, weil sie z.B. vielfache Kristallisationsstufen verlangen und das erwünschte Enantiomere meist in unbefriedigender Ausbeute ergeben. Außerdem werden optisch aktive Amine erfordert, welche in größeren Mengen nicht leicht zugänglich sind.

Die Trennung der Enantiomeren dieser Säuren mit Hilfe der Menthylester weist eine Reihe von Vorteilen auf : So führt das Verfahren in nur wenigen Kristallisationsschritten mit horer Ausbeute zu optisch reinen Verbindungen. Zum anderen ist die Verwendung des preiswerten optisch reinen l- bzw. d-Menthols vorteilhaft, welches leicht wiedergewonnen werden kann. Die bislang nur mühsam erreichbare Bereitstellung von optisch reiner 1R cis-Permethrinsäure führt mit dem erfindungsgemäßen Verfahren auch leicht zu größeren Mengen. Ein besonderer Vorteil ist die selektive Abtrennbarkeit der d- bzw. l-Menthylester der 1R-trans bzw. 1S-trans Permethrinsäure aus dem Gemisch mit den entsprechenden ciskonfigurierten Estern, bzw. die Abtrennung einer Mischung von 1R cis/trans-d-Menthylester (bzw. 1S cis/trans-l-Menthylester) aus dem Gemisch der racem. 1R/S cis/trans-Ester mit d-bzw. l-Menthol.

Die Enantiomerentrennung mit Hilfe der Menthylester ist vor allem zur schnellen Gewinnung größerer Mengen an reinen Enantiomeren geeignet. Aus den Estern können die gewünschten Säuren durch alkalische Verseifung freigesetzt werden.

Die Herstellung der neuen Menthylester gemäß 1 (oben) kann nach an sich bekannten Methoden erfolgen. So wird Verfahren 2a (oben) analog den üblichen Veresterungsmethoden durchgeführt, wie Umsetzung des d- oder l-Menthols

a) mit den freien Säuren in Gegenwart eines wasserbindenden Mittels

b) mit den Säurechloriden in Gegenwart oder Abwesenheit eines Säurefängers oder Lösungsmittels

c) mit den Niedrigalkylestern der Säuren in Gegenwart eines Umesterungskatalysators wie Titantetramethanolat (analog zu der Niederl. Pat 7805738).

Verfahren 2 b wird analog zu dem in DE-OS 2 539 895 beschriebenen Verfahren durchgeführt.

Verfahren 2 c wird analog zu dem in DE-OS 2 723 447 beschriebenen Verfahren durchgeführt.

Verfahren 2 d wird analog zu dem in DE-OS 2 634 663 beschriebenen Verfahren durchgeführt.

Die bei den Verfahren 2a-h verwendeten Ausgangsverbindungen sind bekannt bzw. können im Falle der Verbindungen der Formeln I oder II nach bekannten Verfahren hergestellt werden.

l-Menthol stellt das 1R, 3R, 4S p-Menthanol (-3) und d-Menthol das 1S, 3S, 4R p-Menthanol (-3) dar.

Verwendet man bei Verfahren 2e z.B. 1 Mol racemischen cis-Permenthrinsäurechlorids und 1 Mol d-Menthol als Ausgangsverbindung, so kann man durch einfaches Erwärmen daraus das Gemisch der diastereomeren d-Menthylester erhalten :

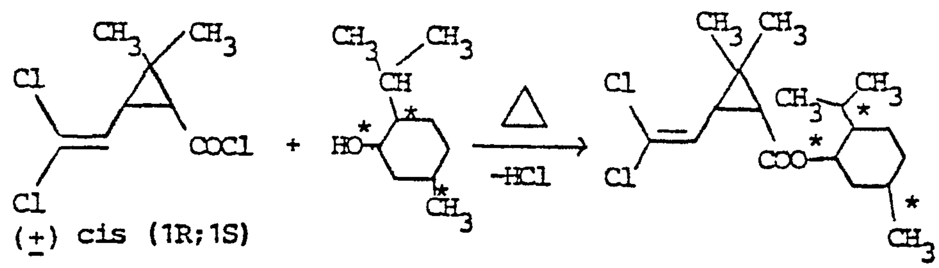


Nach Auflösen in der doppelten Menge Petrolether und Abkühlen auf −10 °C kristallisiert der 1R-cis-Permethrinsäure d-menthylester aus, welcher nach ein bis zwei Kristallisationen aus Petrolether optisch rein ist. Alkalische Verseifung in Methanol bei 120 °C im Autoklaven ergibt nach 5 Std. in 70 % Gesamtausbeute die 1R-cis Permethrinsäure $(\alpha)^D_{20}$ + 27,7° (C = 1, $CHCl_3$) (Lit. DOS 2 4319177 $(\alpha)^D_{20}$ + 27,2).

Das Gemisch der nach den Verfahren 2e-k erhaltenen diastereomeren Menthylester wird in der gleichen oder mehrfachen, vorzugsweise doppelten Menge eines organischen Verdünnungsmittels gelöst. Als solche kommen in Frage unpolare Verdünnungsmittel wie Alkane mit bis zu 10 C-Atomen wie Petrolether, Ligroin, Waschbenzin oder stark polaren Verdünnungsmittel Alkohole, so wie gegebenenfalls sogar wäßrige Alkohole oder Ketone.

Die Abtrennung der schwerer löslichen Enantiomeren erfolgt bei Temperaturen von + 30 bis – 80 °C, vorzugsweise von + 20 bis – 30 °C. Dabei kristallisiert ein Diastereomeres aus, welches bei der Kristallisationstemperatur abgesaugt und zur völligen optischen Reinheit ein oder mehrmals aus dem selben Lösungsmittel umkristallisiert wird.

Die enantiomeren Carbonsäuren werden dann durch alkalische Verseifung der Menthylester mit Alkalihydroxiden in organischen Lösungsmitteln, wie Alkoholen, in Form ihrer Alkalisalze freigesetzt. Diese Verseifung kann zur Beschleunigung der Reaktion bei höheren Temperaturen in höhersiedenden Lösungsmitteln wie z.B. Glykol oder Diphenylether, aber auch in niedrigsiedenden wie Methanol unter Druck durchgeführt werden. Das dabei vollständig wiedergewinnbare unveränderte Menthol ist für weitere Umsetzungen wie beschrieben geeignet.

In einer besonderen Ausführungsform des Verfahrens gemäß 2 g wird aus dem die 4 Diastereomeren enthaltenden Gemisch der d-Menthylester der cis- und trans-Permethrinsäure selektiv der Ester der 1R-trans-Säure in hohen Ausbeuten kristallin abgetrennt. Die Mutterlauge wird sodann mit etwas Photosensibilisator, z.B. Cyclohexanon versetzt und mit einer Ultraviolett-Lichtquelle, wie es in der US-P 3 657 086 und der DE-OS 2 628 477 im Prinzip beschrieben ist, bei Temperaturen zwischen – 70 °C und + 100 °C bestrahlt, bis die optische Drehung sich nicht mehr ändert. Aus der so im Säureteil des Esters isomerisierten racemischen cis/trans Mischung kristallisiert beim Abkühlen erneut der 1R-trans-Ester aus. Die Mutterlauge wird danach wieder photoisomerisiert.

So ist schließlich die gesamte d-Menthylestermischung der ursprünglich racemischen cis/trans-Säure überführbar in den 1R-trans-Ester.

Verwendet man die Ester des natürlichen vorkommenden l-Menthols mit der racemischen cis/trans-Permethrinsäure, so läßt sich durch Abtrennung des schwer löslichen Esters der 1S-trans-Säure und nachfolgende Verseifung der leicht löslichen Bestandteile der Mutterlauge eine Permethrinsäure gewinnen, welche nur noch geringe Reste der 1S-trans-Säure enthält.

Die so erhaltenen reinen optisch aktiven 1R- oder 1S-konfigurierten Cyclopropancarbonsäuren der Formel I werden für die Herstellung von hochwirksamen Insektiziden vom Pyrethroid-Typ verwendet.

Die folgenden Beispiele illustrieren das erfindungsgemäße Verfahren, ohne eine Einschränkung hinsichtlich der Breite seiner Verwendung anzugeben.

Es werden danach z.B. folgende Menthylester erhalten :

A. 3-(2,2-Dichlorvinyl)-2,2-dimethylcyclopropancarbonsäure

| Ester | physik. Eigenschaften |
|---|---|
| (±) (1RS) cis Säure-d Menthylestergemisch | Fp 96° |
| (±) (1RS) trans Säure-d Menthylestergemisch | Fp 101° |
| (±) (1RS) cis/trans Säure-d Menthylestergemisch | $Kp_{0,1}$ 130-140° |
| (±) (1RS) cis Säure-d Menthylester | Fp 96° $[\alpha]^{D}_{13}$ = + 73.6° |
| (–) (1S) cis Säure-d Menthylester | Fp 57° |
| (+) (1R) trans Säure-d Menthylester | Fp 104° = + 69,8° |
| (–) (1S) trans Säure-d Menthylester | |
| (+) (1S) trans Säure-l Menthylester | |
| (–) (1S) trans Säure-l Menthylester | Fp 104° $[\alpha]^{D}_{20}$ –69.3° |

B. 3-(2,2-Dibromvinyl)-2,2-dimethylcyclopropancarbonsäure

| Ester | physik. Eigenschaften |
|---|---|
| (±) (1RS) cis/trans-Säure d Menthylester | |
| (±) (1RS) cis/trans-Säure l Menthylester | |
| (±) (1RS) cis-Säure d Menthylester | |
| (±) (1RS) cis-Säure l Menthylester | |
| (±) (1RS) trans-Säure d Menthylester | $Kp_{0,1}$ 160-170° |
| (±) (1RS) trans-Säure l Menthylester | |
| (+) (1R) cis-Säure d Menthylester | |
| (+) (1R) cis-Säure l Menthylester | |
| (–) (1S) cis-Säure d Menthylester | |
| (–) (1S) cis-Säure l Menthylester | |
| (+) (1R) trans-Säure d Menthylester | Kp 107 $[\alpha]^{D}_{20}$ + 42,1° ($CHCl_3$) |
| (+) (1R) trans-Säure l Menthylester | |
| (–) (1S) trans-Säure d Menthylester | |
| (–) (1S) trans-Säure l Menthylester | Kp 1 070 $[\alpha]^{D}_{20}$ – 42,1° $CHCl_3$ |

Beispiel 1

1,0 Mol (+) cis-3(2,2-Dichlorvinyl)-2,2-dimethylcyclopropancarbonsäurechlorid wird zu einer Mischung von 1,0 Mol d-Menthol ($[\alpha]^D_{20}$ + 47.9 (Ethanol) und 1,0 Mol Triethylamin in 0,7 l Petrolether getropft. Man kocht dann bis zum Verbrauch des Säurechlorides, schüttelt mit Wasser und verdünnter $H_2SO_4$ aus, trocknet und kühlt auf −10 °C ab. Nach einiger Zeit kristallisiert der d-Menthylester der 1R cis-Säure aus, der noch einmal aus der doppelten Menge Petrolether umkristallisiert wird. Ausbeute : 90 %, Fp 96 °C, $[\alpha]^D_{10}$ + 73,6° (C = 1, $CHCl_3$).

Dieser Ester wird mit 1 Mol KOH in 1 l Methanol bei 120° im Autoklaven 6 Std. lang verseift. Nach Abdestillation des Methanols über eine Kolonne wird mit Wasser/Petrolether extrahiert. Die Petroletherphase ergibt das rückgewonne d-Menthol, die wäßrige Lösung wird angesäuert und mit Petrolether extrahiert. Nach Abziehen des Lösungsmittels hält die (+) cis-Säure nach $[\alpha]^D_{20}$ + 27,7° ($CHCl_3$).

Daraus wurde das Säurechlorid hergestellt : $Kp_{0,01}$68° $[\alpha]^D_{20}$ + 17,0° ($CHCl_3$).

Beispiel 2

Analog Beispiel 1 erhielt man mit Hilfe des l-Menthols die opt. reine (−) cis-Saüre. $[\alpha]^D_{20}$ − 27,7° ($CHCl_3$).

Beispiel 3

Analog Beispiel 1 erhält man aus (±) trans-3(2,2-Dichlormethyl)-2,2-dimethyl-cyclopropancarbonsäurechlorid und d-Menthol, die (+) trans-Säure $[\alpha]^D_{20}$ + 36° ($CHCl_3$) Schmelzpunkt des d-Menthylesters der (+) trans-Säure : 92°. $D_{20}$ + 69,5° (C = 1, $CHCl_3$).

Beispiel 4

Die Mutterlauge aus Beispiel 3 wurde wie bei Beispiel 1 beschrieben verseift und ergab nach einmaligem Umkristallisieren aus Petrolether und Abtrennung der racemischen schwer lösliche Anteile opt. reine (−) trans-Säure. $[\alpha]^D_{20}$ − 36,0° ($CHCl_3$). Die NMR-Sprektren der Säure sind identisch mit den beschriebenen.

Beispiel 5

Analog Beispiel 1 wurde mit (+) cis/trans 3-(2,2-Dichlorvinyl)2,2-dimethylcyclopropancarbonsäurechlorid und d-Menthol verfahren. Man erhielt das Diastereomerengemisch $Kp_{0,1}$, 120-130 °C (Ausbeute 95 %). Aus der doppelten Menge Petrolether kristallisierte bei − 20° langsam der d-Menthylester der (+) trans-Saüre in 84 % Ausbeute aus. Nach Kapillar-gaschromatographischer Bestimmung enthält der Ester

zu 83 % Menthyl-Ester der 1R-trans-Säure
zu 7 % Menthyl-Ester der 1S-trans-Säure
zu 5,6 % Menthyl-Ester der 1R-cis-Säure
zu 3,4 % Menthyl-Ester der 1S-cis-Säure

Nach zweimaligem Umkristallisieren war der 1R-trans-Ester-Gehalt auf 94 % gestiegen.
Verseifung mit KOH in Glykol bei 120° ergab nach 5 Std. (+) trans-Säure. $[\alpha]^D_{20}$ + 34°.

Beispiel 6

0,5 Mol (±) cis/trans 3-(2,2-Dichlorvinyl)-2,2-dimethylcyclopropancarbonsäurechlorid wurden bei 100° zu 0,7 Mol l-Menthol getropft. Das sich sogleich entwickelnde HCl-Gas wurde mit einem leichten Stickstoffstrom entfernt. Nach der Destillation erhielt man das Gemisch der Diastereomeren l-Menthylester in 95 % Ausbeute. $Kp_{0,1}$ 120-150°. Nach Kristallisation wie beschrieben erhielt man aus der Mutterlauge einen Ester mit stark verringertem Gehalt an 1S-trans-Ester, der wie in Beispiel 1 verseift wurde.

Beispiel 7

Zu 1 Mol (±) cis/trans 40/60 3-(2,2-Dichlorvinyl)-2,2-dimethylcyclopropancarbonsäureethylester und 1,5 Mol d-Menthol in 40 ml Methanol wurden 5 g Titantetraethylat gegeben. Beim Aufheizen der Suspension destillierte Methanol ab ; die restliche Mischung wurde nun 6 Std. auf 150° erhitzt. Dann fügte man langsam 20 ml abs. Toluol zu und tropfte danach weitere 300 ml Toluol zu, das in der gleichen Geschwindigkeit abdestilliert wurde, bei einer Innentemperatur von 150 °C. Dann wurde bei 0,1 mm destilliert, wobei zuerst nicht umgesetzte Ethylester und Menthol abgetrennt wurden. Die Menthylester

wurden bezogen auf den 56 %igen Umsatz fast quantitativ erhalten. $Kp_{0,1}$ 120-150°. Aus der Lösung in der doppelten Menge Petrolether kristallisierte bei − 5 °C der 1R-trans-Ester mit 85 % Ausbeute.

Beispiel 8

Das nach Beispiel 7 hergestellte d-Menthylestergemisch der racemisch/trans 40/60 Säure wurde in der doppelten Menge Ligroin gelöst. Bei 10° bis 0 °C kristallisierte in 87 % Ausbeute ein Gemisch an Menthylestern aus, das nach GC-Analyse ein Gemisch von 40 % 1R cis- und 60 % 1R-trans Ester darstellte.

Beispiel 9

Die bei Beispiel 7 erhaltene Mutterlauge nach Abtrennen des kristallinen Esters wurde mit 15 g Cyclohexanon versetzt und mit einer Quecksilberhochdrucklampe bei 40° unter Stickstoffschutz bestrahlt. Nach 6 Std. wurde erneut auf − 15 °C abgekühlt, um dann weitere 1R-trans Ester abzusaugen, der zusammen mit den Kristallen aus Beispiel 7 aus Petrolether bei − 5° umkristallisiert wurde. Die Mutterlaugen wurden vereinigt und erneut photoisomerisiert.

Beispiel 10

Analog Beispiel 1 wurde 1R/S trans 3-(2,2-Dibromvinyl)-2,2-dimethylcyclopropancarbonsäurechlorid mit l-Menthol verestert. Siedepunkt des Esters $Kp_{0,1}$ 160°. Ausbeute 73 %. Beim Abkühlen der Petroletherlösung auf − 50° kristallisierte langsam der Ester der 1R-trans-Säure aus. Fp 107°, $[\alpha]^D_{20}$ − 42.1 (C = 1, $CHCl_3$).

NMR-Spektrum ($CHCl_3$) d 6,2 ppm (1), m 4,4-5,0 ppm (1), zu 0,7-2,3 ppm (26).

Beispiel 11 (Vergleichsbeispiel)

Analog Beispiel 1 wurde α-Isopropyl-p-chlorphenylessigsäurechlorid mit l-Menthol zu dem l-Menthylester $Kp_{0,1}$ 180-190° umgesetzt. Es gelang nicht, auf die in den vorgängigen Beispielen beschriebene Methode ein Diastereomeres abzutrennen.

Beispiel 12 (Vergleichsbeispiel)

Analog Beispiel 5 wurde Chrysanthemoylchlorid (cis/trans) mit l-Menthol verestert $Kp_{0,1}$ 130-140°. Es gelang nicht, daraus eines der Diastereomeren aus verschiedenen Lösungsmitteln abzutrennen.

Beispiel 13

Zu 1 Mol 3,3-Dimethylpentensäure in 1 l $CCl_4$ wurden 10 g feuchtes Dibenzoylperoxid gegeben. Anschließend wurde das Wasser herausdestilliert und weitere 5 Std. gekocht. Zwischenzeitlich gab man noch mehr Dibenzoylperoxid zu. Beim Einengen fiel die 6,6,6,4-Tetrachlor-3,3-dimethylhexansäure quantitativ aus. Fp : 118°.

IR-Sprektrum ($cis^{-1}$), KBr-Pressling :

2 300-3 500, 1 700, 1 462, 1 415, 1 405, 1 390, 1 370, 1 340, 1 350, 1 265, 1 235, 1 220, 1 170, 1 120, 1 065, 1 035, 990, 965, 945, 910, 820, 755, 750, 680.

Das NMR-Sprektrum entspricht bezüglich des Säureteils dem in der DE-OS 2 549 895 S. 14 für den Ethylester angegebenen Werten.

0,1 Mol dieser Säure wurde in üblicher Weise mit $SOCl_2$ und etwas DMF in das Säurechlorid überführt und wie in Beispiel 1 Beschrieben mit d-Menthol verestert :

IR-Spektrum des Esters (Film), ($cm^{-1}$) :

2 990, 1 722, 1 450, 1 410, 1 380, 1 360, 1 340, 1 290, 1 260, 1 222-1 190, 1 130, 1 110, 1 666, 1 630, 1 010, 980, 960, 910, 890, 840, 810, 770, 750, 730, 710, 680.

Dieser Ester wurde zu einer Lösung von 0,2 Mol Natrium-d-Menthylat (aus 0,2 Mol d-Menthol und 0,2 Mol NaH hergestellt) in abs. THF gegeben. Bei Raumtemperatur wurde gerührt bis zur Neutralreaktion der Mischung. Nach Absaugen, Einengen und Destillation erhielt man den Permethrinsäure d-Menthylester. Kapilar-Gaschromatographische Analyse ergab das Produktverhältnis :

36,9 % 1R trans-Ester
32,9 % 1S trans-Ester
16,4 % 1R cis-Ester
13,8 % 1S cis-Ester

Durch asymmetrische Induktion erhielt man also einen höheren Gehalt der 1R-trans-Isomeren.

Das Gemisch wurde wie in Beispiel 5 beschrieben mit Petrolether zur Kristallisation gebracht, die Mutterlauge wurde wie in Beispiel 9 photoisomerisiert.

**Ansprüche**

1. Neue d-Menthylester von 1RS cis, 1RS trans, 1RS cis/trans, 1R cis, 1S cis, 1R trans, 1S trans, 1R cis/trans, 1S cis/trans 3-(2,2-Dichlor-vinyl-)2,2-dimethylcyclopropancarbonsäure, neue d-Menthylester von 1RS cis, 1RS trans, 1RS cis/trans, 1R cis, 1S cis, 1R trans, 1S trans, 1R cis/trans, 1S cis/trans 3-(2,2-Dibromvinyl)-2,2-dimethylcyclopropancarbonsäure sowie neue l-Menthylester von 1RS cis, 1RS trans, 1RS cis/trans, 1R cis, 1S cis, 1R trans, 1S trans, 1S cis/trans, 1R cis/trans 3-(2,2-Dibrom-vinyl-)2,2-dimethylcyclopropancarbonsäure sowie neue l-Menthylester von 1R trans, 1S cis/trans, 1R cis/trans, 1S trans 3-(2,2-Dichlor-vinyl)-2,2-dimethylcyclopropancarbonsäure.

2. Verfahren zur Herstellung der neuen Diastereomeren bzw. Diastereomerengemische von 3-(2,2-Dichlorvinyl-)2,2-dimethyl-cyclopropancarbonsäure und 3-(2,2-Dibromvinyl)-2,2-dimethylcyclopropancarbonsäure -d- bzw. l-Menthylester gemäß Anspruch 1, dadurch gekennzeichnet, daß man

a) die entsprechenden Säuren bzw. deren reaktionsfähige Derivate mit d- bzw. l-Menthol umsetzt, oder

b) Menthylester der Formel I

$$X_2C{=}CH{-}\underset{X}{CH}{-}\underset{CH_3}{\overset{CH_3}{C}}{-}CH_2COO\ Men \qquad (I)$$

in welcher

X für Chlor oder Brom steht und

Men für den l- oder d-Menthylrest steht

der cyclisierden Dehydrohalogenierung unterwirft oder

c) Menthylester der Formel II

$$X_2C{=}CH{-}\underset{(CH_3)_2CX}{CH}{-}CH_2{-}COO\ Men \qquad (II)$$

in welcher

X und Men die oben angegebene Bedeutung haben der cyclisierenden Dehydrohalogenierung unterwirft oder

d) Diazoessigsäure -d bzw. -l-menthylester mit 1,1-Dichlor-4-methyl-pentadien-1,3 in Gegenwart von Kupferverbindungen umsetzt oder

e) für den Fall, daß man die d-Menthylester der 1R cis Säuren erhalten will, das Gemisch der entsprechenden 1RS cis-Säuren oder deren reaktionsfähigen Derivaten mit d-Menthol umsetzt und aus Alkanen, Alkoholen, wäßrigen Alkoholen oder Ketonen den schwerer löslichen 1R cis d-Menthylester in üblicher Weise abtrennt, oder

f) für den Fall, daß man die l-Menthylester der 1S cis-Säuren erhalten will, das Gemisch der entsprechenden 1RS cis-Säuren oder deren reaktionsfähigen Derivaten mit l-Menthol umsetzt und aus Alkanen, Alkoholen, wäßrigen Alkoholen oder Ketonen den schwerer löslichen l-Menthylester der 1S cis-Säure in üblicher Weise abtrennt, oder

g) für den Fall, daß man die d-Menthylester der 1R-trans-Säure erhalten will, das Gemisch der entsprechenden 1RS trans-Säuren oder das Gemisch der entsprechenden 1RS cis/trans-Säuren oder deren reaktionsfähigen Derivaten mit d-Menthol umsetzt oder die Reaktionen gemäß b, c oder d (oben) mit den entsprechenden d-Menthylestern durchführt und aus Alkanen, Alkoholen, wäßrigen Alkoholen oder Ketonen den schwerer löslichen d-Menthylester der 1R trans-Säure in üblicher Weise abtrennt oder

h) für den Fall, daß man die l-Menthylester der 1S trans-Säure erhalten will, das Gemisch der entsprechenden 1RS trans-Säuren oder das Gemisch der entsprechenden 1RS cis/trans-Säuren oder deren reaktionsfähigen Derivaten mit l-Menthol umsetzt oder die Reaktionen gemäß b, c oder d (oben) mit den entsprechenden l-Menthylestern durchführt und aus Alkanen, Alkoholen, wäßrigen Alkoholen oder Ketonen den schwerer löslichen l-Menthylester der 1S trans-Säure in üblicher Weise abtrennt.

i) für den Fall, daß man das Gemisch der d-Menthylester der 1R-cis/trans-Säure erhalten will, das Gemisch der entsprechenden 1RS cis/trans-Säuren oder deren reaktionsfähigen Derivaten mit d-Menthol umsetzt oder die Reaktionen gemäß b, c oder d (oben) mit den entsprechenden d-Menthylestern durchführt und aus Ligroin die schwerer löslichen d-Menthylester der 1R-cis/trans-Säure in üblicher Weise abtrennt oder

k) für den Fall, daß man das Gemisch der l-Menthylester der 1R-cis/trans bzw. 1S cis/trans Säure erhalten will, das Gemisch der entsprechenden 1RS cis/trans-Säuren oder deren reaktionsfähigen Derivaten mit l-Menthol umsetzt oder die Reaktionen gemäß b, c oder d (oben) mit den entsprechenden l-Menthylestern durchführt und aus Ligroin den schwerer löslichen l-Menthylester der 1S cis/trans-Säure in üblicher Weise abtrennt und die leichter löslichen 1R cis/trans-Ester weiterverwendet.

3. Verfahren zur Abtrennung von 1R cis 3-(2,2-Dichlorvinyl-)2,2-dimethyl-cyclopropancarbonsäure oder 1R cis 3-(2,2-Dibromvinyl-)2,2-dimethyl-cyclopropancarbonsäure aus dem Gemisch der entsprechenden 1RS cis-Säuren, dadurch gekennzeichnet, daß man das Gemisch dieser Säuren oder ihrer reaktionsfähigen Derivate mit d-Menthol umsetzt, den in Alkanen, Alkoholen, wäßrigen Alkoholen oder Ketonen schwerer löslichen, wäßrigen Alkoholen oder Ketonen schwerer löslichen d-Menthylester der 1R cis-Säure abtrennt und die 1R cis-Säure durch Verseifung des Esters freisetzt.

4. Verfahren zur Abtrennung von 1S cis 3-(2,2-Dichlorvinyl-)2,2-dimethyl-cyclopropancarbonsäure oder 1S cis 3-(2,2-Dibromvinyl)-2,2-dimethyl-cyclopropancarbonsäure aus dem Gemisch der entsprechenden 1RS cis-Säuren, dadurch gekennzeichnet, daß man das Gemisch dieser Säuren oder ihrer reaktionsfähigen Derivate mit l-Menthol umsetzt, den in Alkanen, Alkoholen, wäßrigen Alkoholen oder Ketonen schwerer löslichen l-Menthylester der 1S cis-Säure abtrennt und die 1S cis-Säure durch Verseifung des Esters freisetzt.

5. Verfahren zur Abtrennung von 1R trans 3-(2,2-Dichlorvinyl-)2,2-dimethyl-cyclopropancarbonsäure oder 1R trans 3-(2,2-Dibromvinyl)-2,2-dimethylcyclopropancarbonsäure aus dem Gemisch der entsprechenden 1RS trans-Säuren oder dem Gemisch der entsprechenden 1RS cis/trans-Säuren oder den entsprechenden d-Menthylestergemischen, dadurch gekennzeichnet, daß man das Gemisch dieser Säuren oder ihrer reaktionsfähigen Derivate mit d-Menthol umsetzt, oder die Reaktionen gemäß Anspruch 2b, c oder d (oben) mit den entsprechenden d-Menthylestern durchführt, den in Alkanen, Alkoholen, wäßrigem Alkoholen oder Ketonen schwerer löslichen d-Menthylester der 1R trans-Säure abtrennt und die 1R-trans-Säure durch Verseifung des Esters freisetzt.

6. Verfahren zur Abtrennung von 1S trans 3-(2,2-Dichlorvinyl-)2,2-dimethyl-cyclopropancarbonsäure oder 1S trans 3-(2,2-Dibromvinyl)-2,2-dimethyl-cyclopropancarbonsäure aus dem Gemisch der entsprechenden 1RS trans-Säuren oder dem Gemisch der entsprechenden 1RS cis/trans-Säuren oder den entsprechenden Menthylestergemischen, dadurch gekennzeichnet, daß man das Gemisch dieser Säuren oder ihrer reaktionsfähigen Derivate mit l-Menthol umsetzt oder die Reaktionen gemäß Anspruch 2b, c oder d (oben) mit den entsprechenden Menthylestern durchführt, den in Alkanen, Alkoholen, wäßrigen Alkoholen oder Ketonen schwerer löslichen l-Menthylester der 1S trans-Säure abtrennt und die 1S trans-Säure durch Verseifung des Esters freisetzt.

7. Verfahren zur Abtrennung von 1R cis/trans 3-(2,2-Dichlorvinyl-)2,2-dimethyl-cyclopropancarbonsäure oder 1R cis/trans 3-(2,2-Dibromvinyl)-2,2-dimethylcyclopropancarbonsäure aus dem Gemisch der entsprechenden 1RS cis/trans-Säuren, dadurch gekennzeichnet, daß man das Gemisch dieser Säuren oder ihrer reaktionsfähigen Derivate mit d-Menthol umsetzt, die in Ligroin schwerer löslichen d-Menthylester der 1R cis/trans-Säure abtrennt und die 1R cis/trans Säure durch Verseifung des Esters freisetzt.

8. Verfahren zur Abtrennung von 1R cis/trans 3-(2,2-Dichlorvinyl-)2,2-dimethyl-cyclopropancarbonsäure oder 1R cis/trans 3-(2,2-Dibromvinyl)-2,2-dimethylcyclopropancarbonsäure aus dem Gemisch der entsprechenden 1RS cis/trans-Säuren oder den entsprechenden l-Menthylestergemischen, dadurch gekennzeichnet, daß man das Gemisch dieser Säuren oder ihrer reaktionsfähigen Derivate mit l-Menthol umsetzt, oder die Reaktionen gemäß Anspruch 2b, c oder d (oben) mit den entsprechenden l-Menthylestern durchführt, den in Ligroin schwerer löslichen l-Menthylester der 1S cis/trans-Säure abtrennt und die 1R cis/trans-Säure durch Verseifung des leicht löslichen Esters freisetzt.

**Claims**

1. New d-menthyl esters of 1RS-cis-, 1RS-trans-, 1RS-cis/trans-, 1R-cis-, 1S-cis-, 1R-trans-, 1S-trans-, 1R-cis/trans-, 1S-cis/trans-3-(2,2-dichloro-vinyl)-2,2-dimethylcyclopropanecarboxylic acid, new d-menthyl esters of 1RS-cis-, 1RS-trans-, 1RS-cis/trans-, 1R-cis-, 1S-cis-, 1R-trans-, 1S-trans-, 1R-cis/trans-, 1S-cis/trans-3-(2,2-dibromo-vinyl)-2,2-dimethylcyclopropanecarboxylic acid and new l-menthyl esters of 1RS-cis-, 1RS-trans-, 1RS-cis/trans-, 1R-cis-, 1S-cis-, 1R-trans-, 1S-trans-, 1S-cis/trans-, 1R-cis/trans-3-(2,2-dibromo-vinyl)-2,2-dimethylcyclopropanecarboxylic acid and new l-menthyl esters of 1R-trans-, 1S-cis/trans-, 1R-cis/trans-, 1S-trans-3-(2,2-dichloro-vinyl)-2,2-dimethylcyclopropanecarboxylic acid.

2. Process for the preparation of the new diastereomers or diastereomer mixtures of 3-(2,2-dichloro-vinyl)-2,2-dimethylcyclopropanecarboxylic acid and 3-(2,2-dibromo-vinyl)-2,2-dimethylcyclopropanecarboxylic acid d- and l-menthyl esters according to Claim 1, characterised in that

a) the corresponding acids or reactive derivatives thereof are reacted with d- or l-menthol, or
b) menthyl esters of the formula I

$$X_2C{=}CH{-}\underset{X}{CH}{-}\overset{CH_3}{\underset{CH_3}{C}}{-}CH_2COO\ Men \qquad (I)$$

in which
X represents chlorine or bromine and

Men represents the l- or d-menthyl radical, are subjected to cyclising dehydrohalogenation, or

c) menthyl esters of the formula II

$$\begin{array}{l} X_2C{=}CH{-}\underset{|}{C}H{-}CH_2{-}COO\ Men \\ \quad (CH_3)_2CX \end{array} \qquad (II)$$

in which

X and Men have the meaning indicated above, are subjected to cyclising dehydrohalogenation, or

d) diazoacetic acid d- or l-menthyl ester is reacted with 1,1-dichloro-4-methyl-penta-1,3-diene in the presence of copper compounds or

e) in the case where the d-menthyl esters of the 1R-cis-acids are desired, a mixture of the corresponding 1RS-cis-acids or of reactive derivatives thereof is reacted with d-menthol and the more sparingly soluble 1R-cis-d-menthyl ester is separated off in the customary manner from alkanes, alcohols, aqueous alcohols or ketones, or

f) in the case where the l-menthyl esters of the 1S-cis-acids are desired, a mixture of the corresponding 1RS-cis-acids or their reactive derivatives is reacted with l-menthol and the more sparingly soluble l-menthyl ester of the 1S-cis-acid is separated off in the customary manner from alkanes, alcohols, aqueous alcohols or ketones, or

g) in the case where the d-menthyl esters of the 1R-trans-acid are desired, a mixture of the corresponding 1RS-trans-acids or a mixture of the corresponding 1RS-cis/trans-acids or of reactive derivatives thereof is reacted with d-menthol or the reactions according to b, c or d (above) are carried out with the corresponding d-menthyl esters and the more sparingly soluble d-menthyl ester of the 1R-trans-acid is separated off in the customary manner from alkanes, alcohols, aqueous alcohols or ketones, or

h) in the case where the l-menthyl esters of the 1S-trans-acid are desired, a mixture of the corresponding 1RS-trans-acids or a mixture of the corresponding 1RS-cis/trans-acids or reactive derivatives thereof is reacted with l-menthol or the reactions according to b, c or d (above) are carried out with the corresponding l-menthyl esters and the more sparingly soluble l-menthyl ester of the 1S-trans-acid is separated off in the customary manner from alkanes, alcohols, aqueous alcohols or ketones.

i) in the case where a mixture of the d-menthyl esters of the 1R-cis/trans-acid is desired, a mixture of the corresponding 1RS-cis/trans-acids or of reactive derivatives thereof is reacted with d-menthol or the reactions according to b, c or d (above) are carried out with the corresponding d-menthyl esters and the more sparingly soluble d-menthyl esters of the 1R-cis/trans-acid are separated off in the customary manner from ligroin, or

k) in the case where a mixture of the l-menthyl esters of the 1R-cis/trans- or 1S-cis/trans-acid is desired, a mixture of the corresponding 1RS-cis/trans-acids or of reactive derivatives thereof is reacted with l-menthol or the reactions according to b, c or d (above) are carried out with the corresponding l-menthyl esters and the more sparingly soluble l-menthyl ester of the 1S-cis/trans-acid is separated off from ligroin in the customary manner and the more readily soluble 1R-cis/trans-esters are used for further processing.

3. Process for separating off 1R-cis-3-(2,2-dichloro-vinyl)-2,2-dimethyl-cyclopropanecarboxylic acid or 1R-cis-3-(2,2-dibromo-vinyl)-2,2-dimethyl-cyclopropanecarboxylic acid from a mixture of the corresponding 1RS-cis-acids, characterised in that the mixture of these acids or of reactive derivatives thereof is reacted with d-menthol, the d-menthyl ester of the 1R-cis-acid, which is more sparingly soluble in alkanes, alcohols, aqueous alcohols or ketones, is separated off and the 1R-cis-acid is liberated by saponifying the ester.

4. Process for separating off 1S-cis-3-(2,2-dichloro-vinyl)-2,2-dimethyl-cyclopropanecarboxylic acid or 1S-cis-3-(2,2-dibromo-vinyl)-2,2-dimethyl-cyclopropanecarboxylic acid from a mixture of the corresponding 1RS-cis-acids, characterised in that the mixture of these acids or of reactive derivatives thereof is reacted with l-menthol, the l-menthyl ester of the 1S-cis-acid, which is more sparingly soluble in alkanes, alcohols, aqueous alcohols or ketones, is separated off and the 1S-cis-acid is liberated by saponification of the ester.

5. Process for separating off 1R-trans-3-(2,2-dichloro-vinyl)-2,2-dimethyl-cyclopropanecarboxylic acid or 1R-trans-3-(2,2-dibromo-vinyl)-2,2-dimethyl-cyclopropanecarboxylic acid from a mixture of the corresponding 1RS-trans-acids or from a mixture of the corresponding 1RS-cis/trans-acids or from the corresponding d-menthyl ester mixtures, characterised in that the mixture of these acids or of reactive derivatives thereof is reacted with d-menthol, or the reactions according to Claim 2b, c or d (above) are carried out with the corresponding d-menthyl esters, the d-menthyl ester of the 1R-trans-acid, which is more sparingly soluble in alkanes, alcohols, aqueous alcohols or ketones, is separated off and the 1R-trans-acid is liberated by saponifying the ester.

6. Process for separating off 1S-trans-3-(2,2-dichloro-vinyl)-2,2-dimethyl-cyclopropanecarboxylic acid or 1S-trans-3-(2,2-dibromo-vinyl)-2,2-dimethyl-cyclopropanecarboxylic acid from a mixture of the corresponding 1RS-trans-acids or from a mixture of the corresponding 1RS-cis/trans-acids or from the corresponding menthyl ester mixtures, characterised in that the mixture these acids or of reactive derivatives thereof is reacted with l-menthol or the reactions according to Claim 2b, c or d (above) are

carried out with the corresponding menthyl esters, the l-menthyl ester of the 1S-trans-acid, which is more sparingly soluble in alkanes, alcohols, aqueous alcohols or ketones is separated off and the 1S-trans-acid is liberated by saponifying the ester.

7. Process for separating off 1R-cis/trans-3-(2,2-dichlorovinyl)-2,2-dimethyl-cyclopropanecarboxylic acid or 1R-cis/trans-3-(2,2-dibromo-vinyl)-2,2-dimethylcyclopropanecarboxylic acid from a mixture of the corresponding 1RS-cis/trans-acids, characterised in that the mixture of these acids or of reactive derivatives thereof is reacted with d-menthol, the d-menthyl esters of the 1R-cis/trans-acid, which are more sparingly soluble in ligroin, are separated off and the 1R-cis/trans-acid is liberated by saponifying the ester.

8. Process for separating off 1R-cis/trans-3-(2,2-dichlorovinyl)-2,2-dimethyl-cyclopropanecarboxylic acid or 1R-cis/trans-3-(2,2-dibromo-vinyl)-2,2-dimethylcyclopropanecarboxylic acid from a mixture of the corresponding 1RS-cis/trans-acids or from the corresponding l-menthyl ester mixtures, characterised in that the mixture of these acids or of reactive derivatives thereof is reacted with l-menthol, or the reactions according to Claim 2b, c or d (above) are carried out with the corresponding l-menthyl esters, the l-menthyl ester of the 1S-cis/trans-acid, which is more sparingly soluble in ligroin, is separated off and the 1R-cis/trans-acid is liberated by saponifying the readily soluble ester.

**Revendications**

1. Nouveaux esters de d-menthyle d'acide 1RS cis, 1RS trans, 1RS cis/trans, 1R cis, 1S cis, 1R trans, 1S trans, 1R cis/trans, 1S cis/trans 3-(2,2-dichlorovinyl)-2,2-diméthyl-cyclopropane carboxylique, nouveaux esters de d-menthyle d'acide 1RS cis, 1RS trans, 1RS cis/trans, 1R cis, 1S cis, 1R trans, 1S trans, 1R cis/trans, 1S cis/trans 3-(2,2-dibromovinyl)-2,2-diméthylcyclopropanecarboxylique, ainsi que nouveaux esters de l-menthyle d'acide 1RS cis, 1RS trans, 1RS cis/trans, 1R cis, 1S cis, 1R trans, 1S trans, 1S cis/trans, 1R cis/trans 3-(2,2-dibromovinyl)-2,2-diméthylcyclopropanecarboxylique, ainsi que nouveaux esters de l-menthyle d'acide 1R trans, 1S cis/trans, 1R cis/trans, 1S trans 3-(2,2-dichlorovinyl)-2,2-diméthylcyclopropanecarboxylique.

2. Procédés pour la fabrication des nouveaux diastéréomères ou mélanges de diastéréomères d'esters de d- ou l-menthyle d'acide 3-(2,2-dichlorovinyl)-2,2-diméthylcyclopropanecarboxylique et d'acide 3-(2,2-dibromovinyl)-2,2-diméthylcyclopropanecarboxylique selon la revendication 1, caractérisés en ce que

a) on fait réagir les acides correspondants ou leurs dérivés réactifs avec le d- ou l-menthol, ou bien

b) on soumet à la déshydrohalogénation cyclisante des esters de menthyle de formule I

$$X_2C{=}CH{-}\underset{X}{CH}{-}\overset{CH_3}{\underset{CH_3}{C}}{-}CH_2COO\ Men \qquad (I)$$

dans laquelle

X représente le chlore ou le brome et

Men représente le reste l- ou d-menthyle, ou bien

c) on soumet à la déshydrohalogénation cyclisante des esters de menthyle de formule II

$$X_2C{=}CH{-}\underset{(CH_3)_2CX}{CH}{-}CH_2{-}COO\ Men \qquad (II)$$

dans laquelle X et Men ont la signification indiquée ci-dessus, ou bien

d) on fait réagir des esters de d- ou l-menthyle d'acide diazoacétique avec le 1,1-dichloro-4-méthylpentadiène-1,3 en présence de composés de cuivre, ou bien

e) dans le cas où l'on veut obtenir des esters de d-menthyle des acides 1R cis, on fait réagir le mélange des acides 1RS cis correspondants ou de leurs dérivés réactifs avec le d-menthol et on sépare de la manière habituelle les esters 1R cis de d-menthyle moins solubles à partir d'alcanes, d'alcools, d'alcools aqueux ou de cétones, ou bien

f) dans le cas où l'on veut obtenir les esters de l-menthyle des acides 1S cis, on fait réagir le mélange des acides 1RS cis correspondants ou de leurs dérivés réactifs avec le l-menthol et on sépare de la manière habituelle les esters de l-menthyle de l'acide 1S cis moins solubles à partir d'alcanes, d'alcools, d'alcools aqueux ou de cétones, ou bien

g) dans le cas où l'on veut obtenir les esters de d-menthyle de l'acide 1R trans, on fait réagir le mélange des acides 1RS trans correspondants ou le mélange des acides 1RS cis/trans correspondants ou de leurs dérivés réactifs avec le d-menthol, ou bien on effectue la réaction selon b, c ou d (ci-dessus)

avec les esters de d-menthyle correspondants et on sépare de la manière habituelle les esters de d-menthyle de l'acide 1R trans moins solubles à partir d'alcanes, d'alcools aqueux ou de cétones, ou bien

h) dans le cas où l'on veut obtenir les esters de l-menthyle de l'acide 1S trans, on fait réagir le mélange des acides 1RS trans correspondants ou le mélange des acides 1RS cis/trans correspondants ou de leurs dérivés réactifs avec le l-menthol, ou bien on effectue les réactions selon b, c ou d (ci-dessus) avec les esters de l-menthyle correspondants et on sépare de la manière habituelle les esters de l-menthyle de l'acide 1S trans moins solubles à partir d'alcanes, d'alcools, d'alcools aqueux ou de cétones

i) dans le cas où l'on veut obtenir le mélange des esters de d-menthyle de l'acide 1R cis/trans, on fait réagir le mélange des acides 1RS cis/trans correspondants ou de leurs dérivés réactifs avec le d-menthol, ou bien on effectue la réaction selon b, c ou d (ci-dessus) avec les esters de d-menthyle correspondants et on sépare de la manière habituelle les esters de d-menthyle de l'acide 1R cis/trans moins solubles à partir de la ligroïne, ou bien

k) dans le cas où l'on veut obtenir le mélange des esters de l-menthyle de l'acide 1R cis/trans ou 1S cis/trans, on fait réagir le mélange des acides 1RS cis/trans correspondants ou de leurs dérivés réactifs avec le l-menthol ou bien on effectue la réaction selon b, c ou d (ci-dessus) avec les esters de l-menthyle correspondants et on sépare de la manière habituelle les esters de l-menthyle de l'acide 1S cis/trans moins solubles à partir de la ligroïne et on utilise ultérieurement les esters 1R cis/trans plus solubles.

3. Procédés pour la séparation de l'acide 1R cis 3-(2,2-dichlorovinyl)-2,2-diméthyl-cyclopropanecarboxylique ou de l'acide 1R cis 3-(2,2-dibromovinyl)-2,2-diméthylcyclopropanecarboxylique du mélange des acides 1RS cis correspondants, caractérisés en ce que l'on fait réagir le mélange de ces acides ou de leurs dérivés réactifs avec le d-menthol, on sépare les esters de d-menthyle de l'acide 1R cis moins solubles dans les alcanes, les alcools, les alcools aqueux ou les cétones et on libère l'acide 1R cis par saponification de l'ester.

4. Procédés pour la séparation d'acide 1S cis 3-(2,2-dichlorovinyl)-2,2-diméthylcyclopropanecarboxylique ou d'acide 1S cis 3-(2,2-dibromovinyl)-2,2-diméthylcyclopropanecarboxylique du mélange des acides 1RS cis correspondants, caractérisés en ce que l'on fait réagir le mélange de ces acides ou de leurs dérivés réactifs avec le l-menthol, on sépare les esters de l-menthyle de l'acide 1S cis moins solubles dans les alcanes, les alcools, les alcools aqueux ou les cétones et on libère l'acide 1S cis par saponification de l'ester.

5. Procédés pour la séparation de l'acide 1R trans 3-(2,2-dichlorovinyl)-2,2-diméthylcyclopropanecarboxylique ou de l'acide 1R trans 3-(2,2-dibromovinyl)-2,2-diméthylcyclopropanecarboxylique du mélange des acides 1RS trans correspondants ou du mélange des acides 1RS cis/trans correspondants ou des mélanges d'esters de d-menthyle correspondants, caractérisés en ce que l'on fait réagir le mélange de ces acides ou de leurs dérivés réactifs avec le d-menthol, ou bien on effectue les réactions selon la revendication 2b, c ou d (ci-dessus) avec les esters de d-menthyle correspondants, on sépare les esters de d-menthyle de l'acide 1R trans moins solubles dans les alcanes, les alcools, les alcools aqueux ou les cétones et on libère l'acide 1R trans par saponification de l'ester.

6. Procédés pour la séparation de l'acide 1S trans 3-(2,2-dichlorovinyl)-2,2-diméthylcyclopropanecarboxylique ou de l'acide 1S trans 3-(2,2-dibromovinyl)-2,2-diméthylcyclopropanecarboxylique du mélange des acides 1RS trans correspondants ou du mélange des acides 1RS cis/trans correspondants ou des mélanges d'esters de menthyle correspondants, caractérisés en ce que l'on fait réagir le mélange de ces acides ou de leurs dérivés réactifs avec le l-menthol, ou bien on effectue les réactions selon la revendication 2b, c ou d (ci-dessus) avec les esters de menthyle correspondants, on sépare les esters de l-menthyle de l'acide 1S trans moins solubles dans les alcanes, les alcools, les alcools aqueux ou les cétones et on libère l'acide 1S trans par saponification de l'ester.

7. Procédés pour la séparation de l'acide 1R cis/trans 3-(2,2-dichlorovinyl)-2,2-diméthylcyclopropanecarboxylique ou de l'acide 1R cis/trans 3-(2,2-dibromovinyl)-2,2-diméthylcyclopropanecarboxylique du mélange des acides 1RS cis/trans correspondants, caractérisés en ce que l'on fait réagir le mélange de ces acides ou de leurs dérivés réactifs avec le d-menthol, on sépare les esters de d-menthyle de l'acide 1R cis/trans moins solubles dans la ligroïne et on libère l'acide 1R cis/trans par saponification de l'ester.

8. Procédés pour la séparation de l'acide 1R cis/trans 3-(2,2-dichlorovinyl)-2,2-diméthylcyclopropanecarboxylique ou de l'acide 1R cis/trans 3-(2,2-dibromovinyl)-2,2-diméthylcyclopropanecarboxylique du mélange des acides 1RS cis/trans correspondants ou des mélanges d'esters de l-menthyle correspondants, caractérisés en ce que l'on fait réagir le mélange de ces acides ou de leurs dérivés réactifs avec le l-menthol ou bien on effectue la réaction selon la revendication 2b, c ou d (ci-dessus) avec les esters de l-menthyle correspondants, on sépare les esters de l-menthyle de l'acide 1S cis/trans correspondants moins solubles dans la ligroïne et on libère l'acide 1R cis/trans par saponification de l'ester bien soluble.